# EUROPÄISCHE PATENTSCHRIFT

(11) **EP 0 243 856 B1**
(45) Veröffentlichungstag und Bekanntmachung des Hinweises auf die Patenterteilung: **29.09.1993**
(21) Anmeldenummer: 87105865.7
(22) Anmeldetag: 22.04.1987
(51) Int. Cl.: C12N 15/00

(54) **Verfahren zur Isolierung von Mutanten, mutierten Genen sowie der entsprechenden Wildtyp-Gene**
Process for the isolation of mutants, mutant genes and corresponding wild-type genes
Procédé d'isolement de mutants, de gènes mutés et des gènes du type sauvage correspondants

(30) Priorität: 28.04.1986 DE 3614310
(43) Veröffentlichungstag der Anmeldung: 04.11.1987
(73) Patentinhaber: HOECHST AKTIENGESELLSCHAFT, 65926 Frankfurt am Main (DE)
(72) Erfinder: Wohlleben, Wolfgang, Dr., D-4800 Bielefeld (DE); Muth, Günter, D-4800 Bielefeld (DE); Pühler, Alfred, Prof. Dr., D-4800 Bielefeld (DE)

(56) Entgegenhaltungen:
- JOURNAL OF GENERAL MICROBIOLOGY, Band 131, 1985, pages 1299-1303, SGM, Oxford, GB; A.W. BIRCH et al.: "Temperature-sensitive mutants of the Streptomyces plasmid pIJ702"
- GENE, Band 26, 1983, Seiten 67-78, Elsevier Science Publishers, Amsterdam, NL; K.F. CHATER et al.: "Mutational cloning in Streptomyces and the isolation of antibiotic production genes"
- NATURE, Band 289, Nr. 5793, 1.-8. Januar 1981, Seiten 85-88, Macmillan Journals Ltd, Chesham, Bucks, GB; G.B. RUVKUN et al.: "A general method for site-directed mutagenesis in prokaryotes"
- PROCEEDINGS OF THE NATIONAL ACADEMY OF SCIENCES OF THE USA, Band 79, Nr. 2, Januar 1982, Seiten 520-524, Washington, DC, US; T. LUND et al.: "Isolation of transforming DNA by cosmid rescue"
- BIOTECHNOLOGY, Band 1, Nr. 9, November 1983, Seiten 784-791, New York, US; R. SIMON et al.: "A broad host range mobilization system for in vivo genetic engineering: Transposon mutagenesis in gram negative bacteria"
- GENE, Band 25, 1983, Seiten 29-38, Elsevier Science Publishers, Amsterdam, NL; P.R. ROSTECK et al.: "Selective retention of recombinant plasmids coding for human insulin"

## Beschreibung

Die Erfindung betrifft ein Verfahren, das auf besonders einfache Weise nicht nur die Isolierung von Mutanten erlaubt, sondern auch die Gewinnung des mutierten Gens sowie die Isolierung des entsprechenden nicht mutierten Gens gestattet.

Bei dem erfindungsgemäßen Verfahren wird von einem Plasmid Gebrauch gemacht, das in dem zu mutierenden Ausgangsstamm replizieren kann, Temperatursensitivität aufweist und einen weiteren selektierbaren Marker enthält. Da die Einfuhrung von selektierbaren Markern, beispielsweise Antibiotikaresistenzen, allgemein bekannt ist, bestehen also nur Beschränkungen hinsichtlich des Wirtsbereichs des Ausgangsplasmids.

Es ist bereits bekannt, daß man mit Hilfe von Hydroxylamin temperatursensitive Plasmide herstellen kann (T. Hashimoto et al., J. Bacteriol. 127 (3) 1561 - 1562 (1976); R. Eichenlaub, Isolation of Suppressible Mutations by Hydroxylamine Mutagenesis in Vitro, in: Advanced Mol. Genetics, Herausgeber A. Pühler und K. Timmis; Springer-Verlag, Heidelberg 1984, Seiten 106 - 110; A. W. Birch und J. Cullum, J. Gen. Microbiol. 131, 1299 - 1303 (1985); D.T. Kingsbury et al., Genetics 74 (1973) 17 - 31; R.P. Nowick et al., Proc. Natl. Acad. Sci. USA 79 (1982) 4106 - 4112, A.P. Prozorov et al., Gene 34 (1985) 39 - 46).

Lediglich zur Vereinfachung wird im folgenden die Erfindung anhand von Streptomyceten-Mutationen beschrieben. Die Übertragbarkeit auf andere Mikroorganismen ergibt sich jedoch aus dem Vorhergehenden.

Als Ausgangsplasmide können die bekannten Plasmide pIJ101 oder SLP1.2 (US-Patentschrift 4 360 597), pSG2 (europäische Patentanmeldung mit der Veröffentlichungsnummer 66 701), pSG5 (europäische Patentanmeldungen 158 872 und 158 201) oder pSVH1 (europäische Patentanmeldung 70 522) dienen, die hier jedoch nur beispielhaft aufgeführt sind. Es ist zweckmäßig, diese Ausgangsplasmide vor ihrer Weiterverarbeitung zu verkleinern, wie es beispielsweise in den europäischen Patentanmeldungen 158 201 und 158 872 beschrieben ist. Die Einführung von Markergenen ist an sich bekannt und ebenfalls in den europäischen Patentanmeldungen 158 201 und 158 872 sowie in T. Kieser et al., Mol. Gen. Genet. 185, 223 - 238 (1982) und C.J. Thompson et al., Gene 20, 51 - 62 (1981) beschrieben. Eine eingehende Darstellung befindet sich auch in dem Lehrbuch "Genetic Manipulation of Streptomyces, A Laboratory Manual", The John Innes Foundation, Norwich 1985, in dem auch die anderen Verfahrensschritte ausführlich dargestellt sind.

Geeignete Markergene sind beispielsweise das Thiostrepton-Resistenzgen tsr, das Neomycin-Resistenzgen aphI sowie das Gen für die Melanin-Produktion mel.

Diese so erhaltenen Plasmide mit Markergenen können nun in an sich bekannter Weise so mutiert werden, daß die Replikation temperatursensitiv wird. So wird beispielsweise das Plasmid pIJ702 in die temperatursensitive Replikationsmutante pMT660 überführt (A.W. Birch et al., a.a.0.), die - wie gefunden wurde - in S. lividans bei 39°C verloren geht.

Das erfindungsgemäße Verfahren zur Herstellung von Mutanten ist dadurch gekennzeichnet, daß man aus dem Ausgangsstamm die gesamte DNA isoliert, sie in kurze Fragmente überführt, diese in ein Plasmid integriert, das einen Marker enthält, temperatursensitiv ist und in dem Ausgangs-stamm repliziert. Die erhaltene Hybrid-Population wird in den Ausgangsstamm transferiert und durch Selektion auf den Plasmidmarker überleben nur die Transformanten. Durch Erhöhung der Temperatur über den Schwellenwert des temperatursensitiven Plasmids werden alle autonomen Hybridplasmide eliminiert und durch erneute Selektion auf den Plasmidmarker die Mutanten selektiert, die zur Isolierung des mutierten Gens bzw. zur Isolierung des Wildtyp-Gens, das dem mutierten Gen entspricht, eingesetzt werden.

Andere Aspekte der Erfindung und bevorzugte Ausgestaltungen werden im folgenden näher erläutert bzw. in den Patentansprüchen definiert.

Aus dem Stamm, der einer Mutagenese unterworfen werden soll, isoliert man die gesamte DNA. Diese wird fraktioniert, d.h. zweckmäßig mit geeigneten Restriktionsenzgmen so verdaut, daß u.a. kurze Fragmente entstehen, die weder die Promotorregion noch die Signale für den Translationsstop beeinhalten. Zur Restriktion verwendet man vorteilhaft Restriktionsenzyme, die die DNA häufig schneiden, wie beispielsweise Sau3A oder TagI. Das temperatursensitive Plasmid mit dem Markergen wird nun mit einem geeigneten Restriktionsenzym linearisiert und mit den Restriktionsfragmenten der DNA aus dem zu mutierenden Stamm ligiert. Man erhält dadurch eine Plasmidpopulation mit einer Vielzahl verschiedener kleiner DNA-Fragmente aus dem Ausgangsstamm.

Die Plasmid-Population wird in an sich bekannter Weise in den Ausgangsstamm eingebracht, im Falle von Streptomyceten vorzugsweise durch Polyethylenglykol-induzierte Protoplasten-Transformation.

Die Transformanten, die durch Selektion auf das Markergen erhalten werden, werden bei einer geeigneten, unter dem Schwellenwert des temperatursensitiven Plasmids liegenden Temperatur inkubiert, so daß sich die Transformanten vermehren können. Anschließend erhöht man die Temperatur über den Schwellenwert, was einen Replikationsstop der autonomen Plasmide bedingt. Selektioniert man jetzt auf Anwesenheit des Plasmidmarkers, so erhält man nur Zellen, die Plasmid-DNA in das Chromosom aufgenommen haben. Die Integration der Plasmid-DNA in das Chromosom erfolgt bevorzugt über die klonierte homologe Sequenz des inserierten Fragments und führt im allgemeinen zu einer Inaktivierung des entsprechenden Gens. Damit sind alle Zellen, die oberhalb der Schwellentemperatur wachsen können und den entsprechenden Plasmid-Marker hesitzen. Mutanten.

Die Erfindung erlaubt somit nicht nur eine einfache Erzeugung von Mutanten, sondern auch eine direkte und damit sehr effektive Isolierung. Nach den üblichen Mutageneseverfahren erhält man Mutanten höchstens im Prozentbereich, so daß zur Identifizierung einer Mutante eine große Anzahl nicht mutierter Zellen mit untersucht werden muß. Dieser Aufwand entfällt bei dem erfindungsgemäßen Verfahren. Ein weiterer erheblicher Vorteil der Erfindung ist darin zu sehen, daß durch die Insertion der Plasmid-DNA in das Genom das mutierte Gen physikalisch markiert ist. Dieses Gen ist deshalb leicht zu identifizieren und läßt sich im Gesamtgenom der Mutante durch Hybridisierung erkennen.

Es ist weiterhin möglich, das mutierte Gen durch Desintegration über einen spezifischen Klonierungsschritt direkt aus der DNA der Mutante zu gewinnen. Im Anschluß daran kann das Gen aus einer entsprechenden Genbank des Wildtyps isoliert werden.

Im Gegensatz zu dem in dem obengenannten Lehrbuch beschriebenen "mutational cloning" mit dem Phagenvektoren φC31, dessen Einsatz bisher nur in S. coelicolor beschrieben ist, ist das erfindungsgemäße Verfahren ganz allgemein für Stämme anwendbar, für die ein temperatursensitives Plasmid mit einem Marker zugänglich ist. Außerdem ist die Kopienzahl der Phagengenome pro Chromosom limitiert, und zwar meistens nur auf ein einziges. Setzt man dagegen Plasmide mit hoher Kopienzahl ein, so erhöht man die Rekombinationswahrscheinlichkeit und somit die Mutationsrate. Weiterhin enthält das Phagengenom nur wenige für Klonierungen geeignete Restriktionsschnittstellen, während man bei der Wahl geeigneter Plasmide über eine Vielzahl solcher Stellen verfügen kann. Darüber hinaus können mit den Phagen φC31 nur kurze DNA-Fragmente isoliert werden. Diese Beschränkungen bestehen bei dem erfindungsgemäßen Verfahren nicht, so daß beispielsweise zusammen mit dem mutierten Gen benachbarte unmutierte Gene isoliert werden können. Das erfindungsgemäße Verfahren ist also in jeder Beziehung leistungsfähiger.

In den folgenden Beispielen wird die Erfindung näher erläutert. Prozentangaben beziehen sich hierbei auf das Gewicht, wenn keine anderen Angaben gemacht sind.

### Beispiel 1

### Gesamt-DNA-Isolierung

0,1 g Mycel einer 3 Tage alten, homogenisierten Streptomycetenkultur des Stammes S. ghanaensis (ATCC 14672; US-PS 3 674 866), der kein Melanin produziert (mel⁻), werden im 1,5 ml Eppendorf-Reaktionsgefäß in einer Eppendorf-Zentrifuge 1 min pelletiert und dann einmal mit 0,5 ml TE (10 mM Tris-HCl, 1 mM EDTA (pH 8) mit 10 % Saccharose) gewaschen. Das Pellet wird dann in 0,5 ml Lysozymlösung (0,3 M Saccharose, 25 mM Tris-HCl (pH 8), 25 mM EDTA, 10 mg/ml Lysozym) resuspendiert und 60 min bei 37°C inkubiert. Nach Zugabe von 0,2 ml 5 %iger SDS-Lösung und Durchmischen der Lösung wird diese 10 min bei 65°C inkubiert und danach wieder auf Raumtemperatur abgekühlt. Anschließend gibt man 100 µl Phenol/Chloroform (5 g Phenol, 5 ml Chloroform, 5 mg 8-Hydroxychinolin, 1 ml 0,1 M Tris (pH 8)) zu und mischt kräftig auf einen Schüttler (^{(R)}Vortex), bis die Suspension homogen ist. Danach wird das Gemisch 5 min in einer Eppendorf-Zentrifuge zentrifugiert und die obere, wäßrige Phase in ein neues Reaktionsgefäß überführt. Der DNA-haltigen Lösung wird 70 µl 3 M ungepuffertes Na-acetat und 700 µl Isopropanol zugesetzt. Nach Mischen und 15 minütiger Inkubation bei Raumtemperatur wird die DNA durch Zentrifugation (5 min in Eppendorf-Zentrifuge) pelletiert und der Überstand quantitativ entfernt. Die DNA resuspendiert man in 300 µl TE und inkubiert sie dann 45 min bei 37°C mit 10 µl RNase-Lösung (50 µg RNase/ml H₂0). Die RNase wird durch 100 µl Phenol/Chloroform inaktiviert und die denaturierten Proteine pelletiert (5 min in Eppendorf-Zentrifuge). Die DNA-haltige Lösung wird erneut mit Isopropanol behandelt (Zugabe von 30 µl 3 M Na-acetat und 400 µl Isopropanol, 15 min Inkubation bei Raumtemperatur). Das DNA-Pellet, das man nach der Zentrifugation erhält, wird zweimal mit 70 %igem Ethanol gewaschen und erneut pelletiert. Nach Trocknen nimmt man die DNA in 300 µl TE auf und verwendet sie für die weiteren Schritte.

### Beispiel 2

### Spaltung der Gesamt-DNA mit Sau3A

1 µg DNA wird im Spaltungspuffer (50 nM Tris-HCl (pH 8), 10 mM MgCl₂, 50 mM NaCl) in Gegenwart von 1 unit Sau3A (Hersteller: BRL-Gibco, Karlsruhe) 1 h bei 37°C inkubiert. Die Reaktion wird durch Phenolisierung gestoppt und die DNA durch Ethanolfällung gereinigt.

### Beispiel 3

### Klonierung von Gesamt-DNA-Fragmenten in pMT660 (temperatursensitive Replikationsmutante von pIJ702)

pMT660 (Birch et al., a.a.O.) wird analog Beispiel 2 mit BglII vollständig linearisiert. Die beiden DNA-Proben werden im Spaltungspuffer gemischt, auf 70°C erhitzt und durch Zugabe von Mercaptoethanol (Endkonz. 10 mM) und ATP (0,1 mM) auf die Ligase-Reaktionsbedingungen eingestellt. In Gegenwart von 1 unit T4-DNA-Ligase (Boehringer Mannheim) wird das Gemisch 12 h bei 4°C inkubiert. Anschließend wird das Gemisch in Protoplasten des Ausgangsstammes transformiert und auf Regenerationsplatten ausplattiert. Diese werden nach etwa 20 h mit Weichagar üherschichtet, der soviel Thiostrepton enthält, daß die Endkonzentration in der Platte 50 µg/ml beträgt.

### Beispiel 4

### Erzeugung und Selektion von Mutanten

Die Transformanten werden in einer Schüttelkultur in S-Medium (Hopwood et al., a.a.O.) ca. 36 h bei 28°C inkubiert. Anschließend wird die Temperatur auf 39°C erhöht und nochmals ca. 36 h bei dieser Temperatur inkubiert. Die Kultur wird steril geerntet, in TE + 10 % Saccharose gewaschen und in Vollmedium mit Thiostrepton (20 mg/l) weitere 48 h bei 39°C inkubiert. Anschließend werden die so erzeugten Mutanten ausplattiert und charakterisiert (Inkubation stets bei 39°C).

### Beispiel 5

### Isolierung der mutierten DNA

Aus den Mutanten wird gemäß Beispiel 1 die DNA isoliert, mit einem Restriktionsenzym, das keine Schnittstellen im integrierten Plasmid besitzt, geschnitten und mit T4-DNA-Ligase religiert. Hierdurch wird das integrierte Plasmid, das jetzt das mutierte Gen enthält, als einzige replikationsfähige cyclische DNA gebildet. Nach Retransformation in einen geeigneten Stamm wie S. lividans selektiert man auf Thiostrepton-Resistenz und isoliert aus den Transformanten das Plasmid, das das mutierte Gen trägt.

## Patentansprüche

1. Verfahren zur Isolierung eines mutierten Gens aus Mutanten, dadurch gekennzeichnet, daß man zunächst zur Herstellung der Mutanten die gesamte DNA aus dem Ausgangsstamm isoliert, sie in kurze Fragmente überführt, diese in ein Plasmid integriert, das einen Marker enthält, temperatursensitiv ist und in dem Ausgangsstamm repliziert, die erhaltene Hybrid-Population in den Ausgangsstamm transformiert, durch Selektion auf den Marker die Transformanten selektiert, durch Erhöhung der Temperatur über den Schwellenwert des temperatursensitiven Plasmids die freien Hybridplasmide elimiert, durch erneute Selektion auf den Marker die Mutanten selektiert, anschließend die Gesamt-DNA isoliert, mit einem Restriktionsenzym, das keine Schnittstellen im integrierten Plasmid besitzt, schneidet, dann mit DNA Ligase religiert, nach Retransformation in einen geeigneten Wirt auf den genannten Marker selektiert, aus den Transformanten Plasmide mit markiertem Genisoliert, und anschließend besagte Plasmide zur Isolierung mutierter Gene einsetzt.

2. Verfahren nach Anspruch 1 , dadurch gekennzeichnet, daß der Ausgangsstamm der Gattung Streptomyces angehört.

3. Verfahren nach Anspruch 1 oder 2, dadurch gekennzeichnet, daß das Plasmid als Marker eine Antibiotika-Resistenz enthält.

4. Verfahren nach Anspruch 1, 2 oder 3, dadurch gekennzeichnet, daß man ein Plasmid einsetzt, das durch Behandlung mit Hydroxylamin temperatursensitiv gemacht wurde.

5. Verwendung eines nach einem oder mehreren der Ansprüche 1 bis 4 erhaltenen mutierten Gens zur Isolierung des Wildtyp-Gens, das dem mutierten Gen entspricht.

## Claims

1. A process for the isolation of a mutated gene from mutants, which comprises initially , for the preparation of the mutants, the total DNA being isolated from the original strain and converted into short fragments, and the latter being integrated into a plasmid which contains a marker, is temperature-sensitive and replicates in the original strain, the resulting hybrid population being transformed into the original strain, the transformants being selected by selection for the marker, the free hybrid plasmids being eliminated by raising the temperature above the threshold of the temperature-sensitive plasmid, and the mutants being selected by renewed selection for the marker, and subsequently the total DNA being isolated, cut with a restriction enzyme which has no cleavage sites in the integrated plasmid and then religated with DNA ligase, carrying out selection, after retransformation into a suitable host, for the abovementioned marker, plasmids having the gene with marker being isolated from the transformants, and subsequently said plasmids being employed for the isolation of mutated genes.

2. The process as claimed in claim 1, wherein the original strain belongs to the genus Streptomyces.

3. The process as claimed in claim 1 or 2, wherein the plasmid contains an antibiotic resistance as marker.

4. The process as claimed in claim 1, 2 or 3, wherein use is made of a plasmid which has been made temperature-sensitive by treatment with hydroxylamine.

5. The use of a mutated gene obtained as claimed in one or more of claims 1 to 4 for the isolation of the wild type gene which corresponds to the mutated gene.

## Revendications

1. Procédé d'isolement d'un gène muté à partir de mutants, caractérisé en ce que, d'abord pour fabriquer les mutants, on isole l'ADN total à partir de la souche initiale, on le transforme en fragments courts, on intègre ces derniers dans un plasmide, qui contient un marqueur, est sensible à la température et dans lequel la souche initiale se réplique, on transforme dans la souche initiale la population hybride obtenue, on sélectionne les transformants par sélection sur le marqueur, on élimine les plasmides hybrides libres par chauffage du plasmide sensible à une température supérieure au seuil, on sélectionne les mutants par une nouvelle sélection sur le marqueur, ensuite on isole l'ADN total, on le coupe avec une enzyme de restriction qui ne possède pas de sites de clivage dans le plasmide intégré, puis on le ressoude à l'aide d'une ADN ligase, on le sélectionne sur ledit marqueur après retransformation dans un hôte approprié, on isole à partir des transformants les plasmides porteurs du gène marqué et on utilise ensuite lesdits plasmides pour isoler les gènes mutés.

2. Procédé selon la revendication 1, caractérisé en ce que la souche initiale appartient au genre Streptomyces.

3. Procédé selon la revendication 1 ou 2, caractérisé en ce que le plasmide contient, comme marqueur, un marqueur résistant aux antibiotiques.

4. Procédé selon une des revendications 1, 2 ou 3, caractérisé en ce qu'on utilise un plasmide qui est rendu sensible à la température par traitement avec de l'hydroxylamine.

5. Utilisation d'un gène muté obtenu selon une ou plusieurs des revendications 1 à 4, pour l'isolement du gène de type sauvage correspondant au gène muté.
